# FASCICULE DE BREVET EUROPEEN

(11) **EP 4 408 489 B1**
(45) Date de publication et mention de la délivrance du brevet: **17.12.2025**
(21) Numéro de dépôt: 22792843.9
(22) Date de dépôt: 22.09.2022
(51) Int. Cl.: A61L 11/00, A61L 2/22, A61L 2/18, B09B 3/00, B65F 1/10

(54) **BOÎTE DESTINÉE À RECEVOIR DES DÉCHETS, EN PARTICULIER DES DÉCHETS MÉDICAUX**
BEHÄLTER ZUR AUFNAHME VON ABFÄLLEN, INSBESONDERE MEDIZINISCHEN ABFÄLLEN
BOX INTENDED TO RECEIVE WASTE, IN PARTICULAR MEDICAL WASTE

(30) Priorité: 30.09.2021 FR 2110307
(43) Date de publication de la demande: 07.08.2024
(73) Titulaire: Europe Metal Concept (EMC), 13690 Graveson (FR)
(72) Inventeur: PIAZZA, Louis, 13630 Eyragues (FR)
(74) Mandataire: Germain Maureau
(86) Numéro de dépôt international: PCT/FR2022/051781
(87) Numéro de publication internationale: WO 2023/052709

(56) Documents cités:
- WO-A1-2012/125666
- WO-A2-2010/146004
- JP-A- 2000 279 928
- US-B1- 7 878 359

## Description

L'invention concerne une boîte destinée à recevoir des déchets, en particulier des déchets médicaux et un procédé de recyclage d'une boîte. On connaît, par exemple, le document WO 2012/125666A1, qui décrit des boîtes pour recevoir des déchets.

De tels déchets peuvent typiquement être issus d'activités de diagnostic ou de traitement dans les domaines de la médecine humaine et vétérinaire, et peuvent présenter un risque infectieux du fait qu'ils contiennent des micro-organismes susceptibles de causer des maladies chez l'homme ou chez d'autres organismes vivants. Il peut s'agir de pansements, seringues, bistouris, tubulures, etc.

Ces déchets, qui peuvent être appelés DASRI (déchets d'activités de soins à risques infectieux), font l'objet d'une réglementation particulière. En particulier, le stockage et le transport de ces déchets doit s'effectuer dans des boîtes adaptées ; puis ces déchets ainsi que la boîte qui les contient sont éliminés par incinération ou par enfouissement, dans une filière différente de celle des déchets ménagers.

D'une part, l'élimination de ces déchets requiert des procédés relativement lourds et coûteux, afin de garantir qu'ils ne puissent plus présenter de risque infectieux une fois éliminés. A titre d'exemple, la température d'incinération de ces déchets est nettement plus élevée que celle des déchets ménagers.

D'autre part, la nécessité d'éliminer également la boîte, qui présente elle aussi potentiellement un risque infectieux, engendre un très grand volume d'éléments jetés car inutilisables, ce qui n'est évidemment pas souhaitable.

La présente invention vise à remédier aux inconvénients mentionnés ci-dessus.

A cet effet, l'invention concerne une boîte destinée à recevoir des déchets, en particulier des déchets médicaux, comprenant :
- un bac comportant une paroi périphérique, une paroi de fond, et une ouverture opposée à la paroi de fond ;
- une platine fixée au bac, couvrant sensiblement l'ouverture du bac, et comportant au moins une fenêtre pour l'introduction de déchets dans le bac ;
- un couvercle monté sur la platine de façon mobile entre une position ouverte, dans laquelle la fenêtre de la platine est accessible, et une position fermée, dans laquelle le couvercle obture ladite fenêtre.

Par « la platine couvre sensiblement l'ouverture du bac », on entend que la platine couvre entièrement ladite ouverture, à l'exception de la zone où se situe la fenêtre pour l'introduction de déchets dans le bac.

Selon une définition générale de l'invention, la boîte comprend en outre une cartouche apte à contenir un fluide de désinfection des déchets, la cartouche étant montée dans le bac et comportant :
- une enveloppe ;
- un trou d'expulsion du fluide ;
- un actionneur mobile par rapport à l'enveloppe entre une position inactive dans laquelle le fluide ne peut pas être expulsé de la cartouche et une position active dans laquelle du fluide peut être expulsé par le trou d'expulsion ;
et le couvercle comprend des moyens d'activation de l'actionneur de la cartouche.

La boîte est configurée pour que, lorsque le couvercle est en position ouverte, l'actionneur soit en position inactive, et pour que le passage du couvercle à la position fermée provoque le passage de l'actionneur à la position active par l'action des moyens d'activation.

De plus, la cartouche est montée dans le bac de façon inamovible, et la boîte comporte des moyens de rechargement de la cartouche en fluide, les moyens de rechargement étant ménagés sur la cartouche et/ou sur le bac et étant accessibles depuis l'extérieur de la boîte.

Par « inamovible », on entend que la cartouche est fixée à demeure dans le bac et ne peut en être désolidarisée dans des conditions normales d'usage, sauf bien entendu à mettre en œuvre une force importante ou à utiliser des outils.

L'invention permet donc de désinfecter les déchets contenus dans le bac, et ce de façon automatique lorsque le praticien ferme le couvercle, ce qui correspond au protocole d'utilisation de la boîte une fois qu'elle est remplie, avant sa prise en charge par une entreprise spécialisée.

Les déchets ne présentent alors plus de risque infectieux, et peuvent être traités dans la filière des déchets ménagers, c'est-à-dire selon des procédés moins contraignants et moins onéreux. En outre, le bac, une fois vidé de ces déchets désinfectés, peut être réutilisé pour former une nouvelle boîte, de préférence en étant préalablement nettoyé et désinfecté. On peut ainsi réaliser une réduction très significative du volume total de déchets.

En pratique, le passage de l'actionneur à la position active peut s'effectuer en toute fin de mouvement de fermeture du couvercle. La configuration de la boîte permettant que les moyens d'activation ménagés sur le couvercle agissent sur l'actionneur peut concrètement correspondre à une configuration adaptée à cet effet de la cartouche et du couvercle (en particulier la structure de ces deux éléments et leur agencement mutuel), comme cela est décrit ci-après.

Selon une réalisation possible, les moyens d'activation comprennent un ergot faisant saillie sensiblement orthogonalement de la face intérieure du couvercle. En outre, la platine comprend un orifice qui, lorsque le couvercle est en position fermée, est situé en regard de l'ergot du couvercle et de l'actionneur de la cartouche, de sorte que l'ergot puisse coopérer avec l'actionneur pour le faire passer à sa position active.

En variante, le couvercle pourrait être dépourvu d'ergot, et présenter une face inférieure sensiblement plane qui vient coopérer avec un actionneur en saillie au-dessus de la platine.

Selon différentes possibilités, l'ergot fait saillie sous la platine, ou l'actionneur fait saillie au-dessus de la platine, ou une face de contact entre l'ergot et l'actionneur lors de leur coopération est située dans l'épaisseur de la platine.

En position fermée du couvercle, l'ergot peut être au moins en partie logé dans l'orifice de la platine. De préférence, l'orifice de la platine présente des dimensions légèrement supérieures à celles de l'ergot, afin d'assurer un guidage et un positionnement approprié de l'ergot, en particulier par rapport à l'actionneur.

Selon une réalisation possible, l'actionneur comprend un embout monté en partie supérieure de la cartouche de façon mobile en translation verticale, entre une position haute, correspondant à la position inactive, et une position basse, correspondant à la position active, l'embout étant agencé en regard d'un orifice de la platine de sorte à pouvoir coopérer avec les moyens d'activation ménagés sur le couvercle.

Le trou d'expulsion du fluide peut être ménagé dans l'embout, l'embout formant ainsi une partie d'un système de type valve d'aérosol.

L'embout peut être engagé au moins partiellement dans l'orifice de la platine en positon fermée du couvercle. Par exemple, l'orifice de la platine présente des dimensions légèrement supérieures à celles de l'embout, pour assurer un guidage de celui-ci.

De préférence, l'orifice de la platine est distinct de la fenêtre par laquelle les déchets peuvent être introduits.

On peut prévoir que, en position inactive, l'embout soit situé sous la face supérieure de la platine. Une telle disposition permet d'empêcher un actionnement accidentel de l'embout à un moment inapproprié. Il pourrait en conséquence ne plus y avoir suffisamment de fluide désinfectant dans la cartouche pour désinfecter efficacement les déchets une fois le couvercle fermé. De façon concrète, la face supérieure de l'embout peut affleurer la face supérieure de la platine, être située dans l'épaisseur de la platine, ou être située sous la face inférieure de la platine.

Selon une variante n'appartenant pas à l'invention, la cartouche pourrait être montée dans le bac de façon amovible. Dans ce cas, une fois vide, la cartouche pourrait être remplacée par une cartouche pleine.

Par exemple, les moyens de rechargement comportent une pièce qui est montée dans un trou du bac, en regard de la cartouche, de façon mobile entre une position ouverte, permettant l'entrée dans la cartouche du fluide de désinfection des déchets, et une position fermée assurant l'étanchéité audit fluide entre l'intérieur et l'extérieur du bac. La pièce peut être sollicitée vers sa position fermée par un ressort. Le passage vers la position ouverte peut être obtenu par la tête d'un système d'injection de fluide qui est insérée avec étanchéité dans ladite pièce, et qui exerce une poussée sur ladite pièce à l'encontre de la force exercée par le ressort.

De façon alternative, le rechargement pourrait s'effectuer par l'embout.

La cartouche peut être disposée dans le bac à proximité de ou contre la paroi périphérique du bac. Ceci permet notamment de laisser un volume important pour la réception des déchets. Avec cette disposition, et afin de ne pas interférer avec la cartouche, on peut prévoir que la fenêtre d'introduction des déchets soit sensiblement centrale sur la platine. Un avantage de disposer la cartouche contre la paroi périphérique du bac - c'est-à-dire sans espace entre la cartouche et la paroi périphérique du bac - est de limiter au sein du bac les espaces restreints difficiles à désinfecter.

Selon une réalisation possible, à l'état initial, c'est-à-dire avant le montage ou au cours d'une étape de montage des différents constituants de la boîte, l'enveloppe forme un volume ouvert. De plus, la boîte comporte au moins une paroi de fermeture qui, à l'état prêt à l'emploi, coopère avec l'enveloppe pour former un volume fermé apte à contenir le fluide. Par « état prêt à l'emploi », on entend l'état dans lequel la boîte est fournie au praticien pour qu'il puisse y jeter les déchets. La boîte est donc montée, en particulier avec la cartouche mise en place dans le bac et la platine montée sur le bac. Le fait d'avoir à l'état prêt à l'emploi une cartouche intégrée au bac est très avantageux. En effet, cela limite considérablement les interstices où des agents infectieux pourraient se loger , et où il serait difficile de les neutraliser. Une telle disposition permet de répondre à une problématique de moulage du volume fermé de la cartouche.

La paroi de fermeture peut être formée au moins en partie par une paroi du bac. Par exemple, la paroi de fermeture peut être formée par une portion de la paroi de fond et de la paroi périphérique du bac, qui peuvent former respectivement au moins une partie de la paroi de fond et de la paroi périphérique de l'enveloppe de la cartouche. En variante, ou en complément, l'enveloppe peut être réalisée en plusieurs pièces qui viennent s'assembler, tel que deux demi-coquilles. La paroi de fermeture peut comporter ou être constituée de l'une de ces pièces (par exemple de l'une de ces pièces et d'une partie d'une paroi du bac).

De façon alternative, la paroi de fermeture pourrait être une paroi distincte de l'enveloppe et du bac, à l'état initial. Par exemple, l'enveloppe de la cartouche peut être un cylindre à l'extrémité supérieure ouverte, la paroi de fermeture étant un disque venant obturer cette extrémité supérieure.

Selon un mode de réalisation, l'enveloppe est une pièce distincte du bac, à l'état initial, qui comporte une ouverture inférieure et une ouverture latérale, ladite enveloppe venant se fixer avec étanchéité contre la face intérieure du bac, de sorte que la paroi de fond du bac forme le fond de la cartouche et que la paroi périphérique du bac ferme l'ouverture latérale de l'enveloppe.

On peut prévoir que la zone de la paroi de fond du bac qui forme le fond de la cartouche et la zone de la paroi périphérique du bac qui ferme l'ouverture latérale de l'enveloppe forment une surépaisseur locale, vers l'intérieur du bac, par rapport aux zones adjacentes de la paroi de fond et de la paroi périphérique du bac, respectivement, permettant un guidage et un emboîtement de l'enveloppe contre le bac.

Selon un autre mode de réalisation, la boîte comprend au moins deux portions initialement distinctes, chaque portion constituant une portion de bac et une portion d'enveloppe et étant de préférence réalisée d'une seule pièce par moulage, lesdites deux portions étant assemblées avec étanchéité pour former le bac et la cartouche intégrée au bac. Ces deux portions peuvent être sensiblement des moitiés, de part et d'autre d'un plan vertical médian de la boîte. L'assemblage peut être effectué par thermosoudage.

La zone de la paroi de fond du bac qui forme le fond de la cartouche peut posséder une partie ayant une forme bombée vers l'intérieur du bac, favorisant l'expulsion appropriée du fluide de désinfection.

Selon un deuxième aspect, l'invention concerne un procédé de recyclage d'une boîte telle que précédemment décrite, le couvercle étant en position fermée. Le procédé comprend les étapes suivantes :
- désolidariser du bac l'ensemble comprenant la platine et le couvercle ;
- vider les déchets contenus dans le bac ;
- nettoyer et désinfecter le bac ;
- recharger la cartouche en fluide de désinfection, par lesdits moyens de rechargement, depuis l'extérieur de la boîte ;
- monter à nouveau sur le bac l'ensemble comprenant la platine et le couvercle.

Les déchets peuvent être vidés comme des déchets ménagers puisqu'ils ont été désinfectés à la fermeture du couvercle.

A l'issue de ce procédé, et une fois le couvercle placé en position ouverte, la boîte peut à nouveau être utilisée par un praticien.

La boîte est généralement conçue pour qu'un praticien ne puisse pas désolidariser la platine du bac ni rouvrir le couvercle une fois qu'il a été fermé. Ces opérations ne peuvent être réalisées que par une entreprise spécialisée, typiquement au moyen de machines adaptées.

On décrit à présent, à titre d'exemples non limitatifs, plusieurs modes de réalisation possibles de l'invention, en référence aux figures annexées :
La figure 1 est une vue en perspective d'une boîte selon un mode de réalisation de l'invention, le couvercle étant en position fermée ;
La figure 2 est une vue en perspective de la boîte de la figure 1, le couvercle étant en position ouverte ;
La figure 3 est une vue de détail de la figure 3, montrant le verrouillage du couvercle sur la platine ;
La figure 4 est une vue en perspective de la boîte de la figure 1, montrant une cartouche à l'intérieur du bac ;
La figure 5 est une vue schématique en coupe verticale transversale de la platine et du couvercle de la boîte ;
La figure 6 est une vue schématique et partielle de la boîte, en coupe verticale longitudinale ;
La figure 7 est une vue de dessus de la boîte ouverte, montrant l'intérieur du bac en l'absence de cartouche, selon un mode de réalisation ;
La figure 8 est une vue de détail de la paroi de fond du bac de la figure 7, selon une section verticale longitudinale ;
La figure 9 est une vue en perspective d'une cartouche selon un mode de réalisation ;
La figure 10 est une vue en perspective, selon un autre angle de vue, de la cartouche de la figure 9 ;
La figure 11 est une vue de dessus de la boîte ouverte, montrant l'intérieur du bac de la figure 6 équipé de la cartouche des figures 9 et 10 ;
La figure 12 illustre une étape de fabrication du bac d'une boîte selon un autre mode de réalisation ;
La figure 13 est une vue de détail en coupe verticale longitudinale illustrant des moyens de rechargement de la cartouche en fluide, à l'état inactif ;
La figure 14 est une vue similaire à la figure 13, les moyens de rechargement de la cartouche en fluide étant à l'état actif, lors du rechargement.

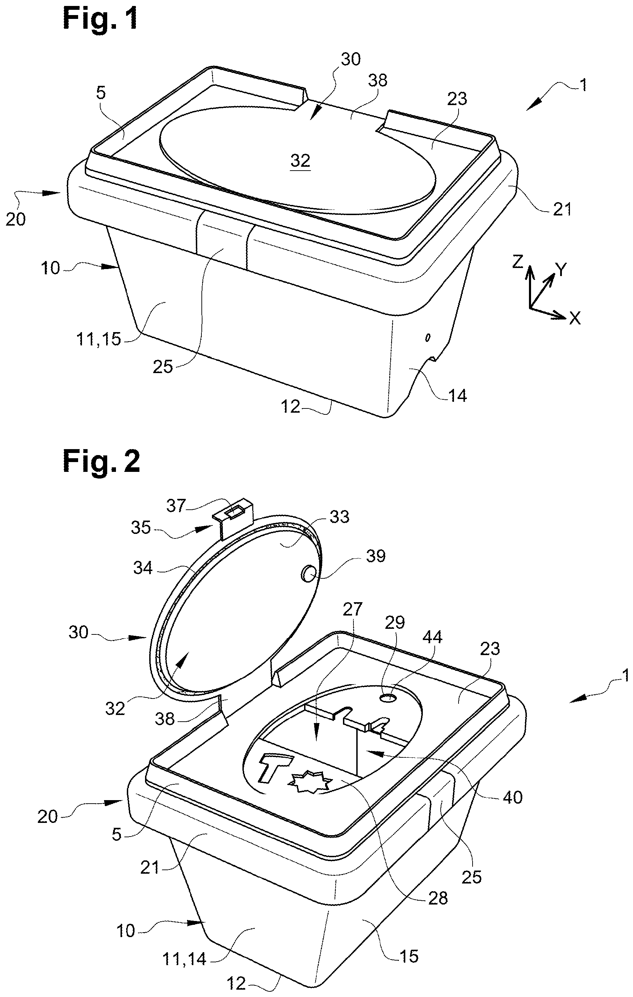

La figure 1 représente une boîte 1 selon un mode de réalisation de l'invention, qui est typiquement destinée à recevoir des déchets médicaux présentant un risque infectieux.

La boîte 1 comprend un bac 10 qui comporte une paroi périphérique 11, une paroi de fond 12, et une ouverture 13 opposée à la paroi de fond 12. Le bac 10 peut être sensiblement parallélépipédique, éventuellement avec une paroi périphérique 11 qui s'évase depuis la paroi de fond 12 en direction de l'ouverture 13. Ainsi, selon cet exemple de réalisation, la paroi de fond 12 est sensiblement rectangulaire et globalement plane, et la paroi périphérique 11 comporte deux petits panneaux 14 en regard et deux grands panneaux 15 en regard. Le bac 10 peut être réalisé par moulage d'une matière plastique.

La boîte 1 est décrite lorsque la paroi de fond 12 repose sur une surface horizontale, c'est-à-dire dans la position illustrée sur la figure 1 notamment. On définit la direction Z comme la direction orthogonale à la paroi de fond 12, et donc verticale dans la position décrite, la direction longitudinale X comme la direction orthogonale à Z et aux petits panneaux 14, et la direction transversale Y comme le direction orthogonale à X et à Z. Cette position est celle dans laquelle la boîte 1 est utilisée par un praticien. Toutefois, on comprend que la boîte peut prendre d'autres orientations dans l'espace, notamment lors de son transport.

Les termes « dessus », « haut », « bas », « supérieur », « inférieur » et analogue sont définis en relation avec la direction Z.

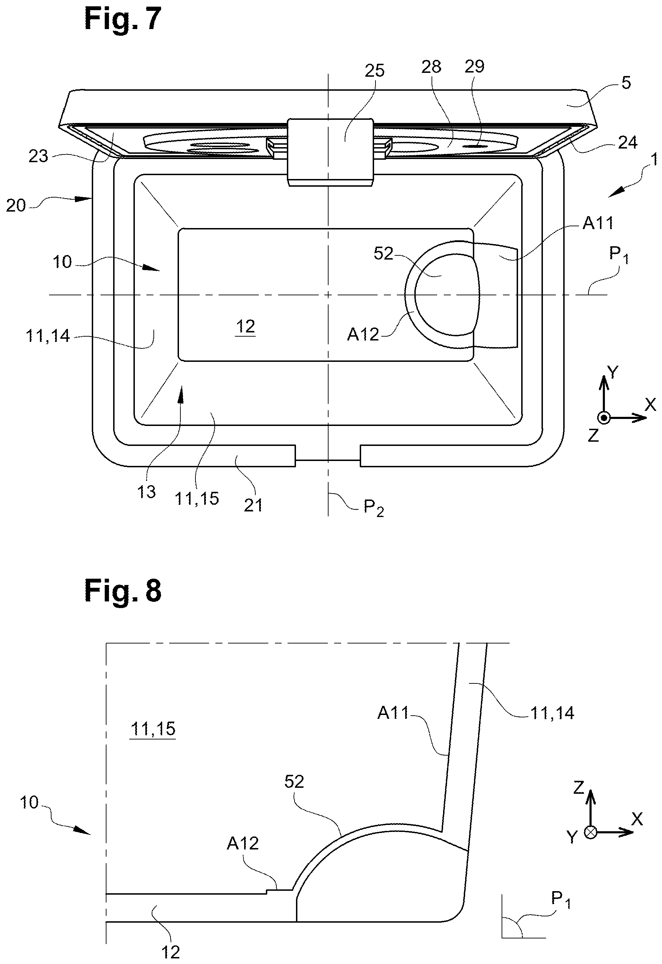

La boîte 1 présente un plan vertical longitudinal médian P1 et un plan vertical transversal médian P2, comme on le voit notamment sur la figure 7.

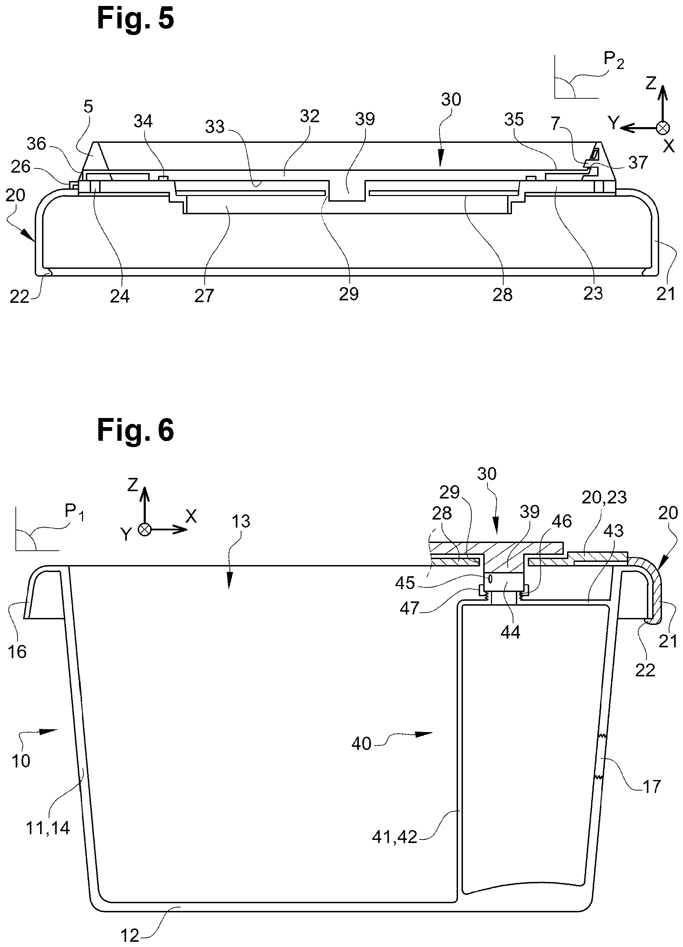

A sa partie supérieure, la paroi périphérique 11 peut présenter un rebord 16 dirigé vers l'extérieur et vers le bas, qui peut s'étendre sur toute la périphérie de l'ouverture 13 (voir figure 5, qui est une coupe selon le plan P2).

La boîte 1 comprend également une platine 20 qui est fixée au bac 10. La platine 20 peut comporter un cadre 21 qui vient se placer autour de l'ouverture 13, par exemple sur le rebord 16. De préférence, la platine 20 est verrouillée sur le bac 10, de sorte que le praticien ne puisse pas l'ôter, seule une entreprise spécialisée dans le traitement des déchets en question pouvant désolidariser la platine 20 du bac 10 par des moyens spécifiques. A cet effet, le cadre 21 peut être pourvu à sa partie inférieure d'un bourrelet 22 intérieur venant s'emboîter sous le rebord 16 du bac 10.

La platine 20 peut en outre comporter une plaque 23 qui est montée sur le cadre 21. Lors de l'utilisation de la boîte 1, la plaque 23 couvre sensiblement l'ouverture 13 du bac 10, la plaque 23 pouvant donc être sensiblement rectangulaire. Dans cette position d'utilisation, ou position fermée de la plaque 23, la face inférieure de la plaque 23 peut reposer avec étanchéité sur le bord supérieur de la paroi périphérique 11 du bac 10, par exemple sur le rebord 16. L'étanchéité peut être assurée par l'interposition d'un joint périphérique 24, par exemple monté sur la plaque 23. Comme illustré notamment sur la figure 7, la plaque 23 peut comporter une languette 25 apte à coopérer avec le cadre 21, pour assurer le verrouillage de la plaque 23 en position fermée sur le cadre 21.

La plaque 23 peut être montée pivotante par rapport au cadre 21, par exemple via une charnière 26 d'axe parallèle à la direction X, située le long d'un grand panneau 15 du bac 10, à l'opposé de la languette 25. La platine 20 peut être réalisée d'une seule pièce par moulage d'une matière plastique, la charnière 26 étant formée d'une bande mince reliant le cadre 21 et la plaque 23.

La platine 20 comporte au moins une fenêtre 27 pour l'introduction de déchets dans le bac 10. La fenêtre 27 peut être disposée de façon sensiblement centrale par rapport à l'ouverture 13 du bac 10 ; elle peut être sensiblement rectangulaire, selon une réalisation possible.

La fenêtre 27 peut être ménagée dans la plaque 23. La plaque 23 peut comporter une zone en dépression 28, c'est-à-dire une zone située plus bas que le reste de la plaque 23 lorsque la plaque 23 est en position fermée, dans laquelle est ménagée cette fenêtre 27. Comme on le voit sur la figure 2, la zone en dépression 28 peut s'étendre au-delà de la fenêtre 27, selon la direction X et/ou Y. Elle présente ici une forme ovale, ce qui n'est pas limitatif.

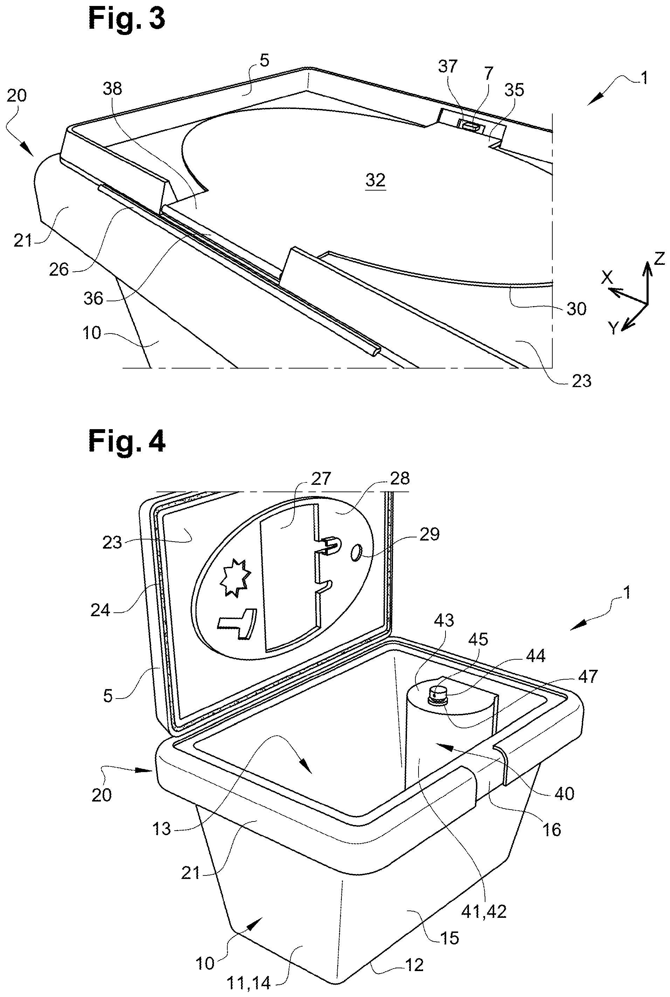

La boîte 1 comprend en outre un couvercle 30 qui est monté sur la platine 20 de façon mobile entre une position ouverte (figures 2 et 4), dans laquelle la fenêtre 27 est accessible, et une position fermée (figures 1 et 3), dans laquelle le couvercle 30 obture cette fenêtre 27.

Le couvercle 30 peut être monté pivotant par rapport à la platine 20, par exemple via une charnière 36 d'axe parallèle à la direction X, qui peut être adjacente à la charnière 26 comme on le voit sur la figure 3. Le couvercle 30 et la platine 20 peuvent être réalisés d'une seule pièce par moulage d'une matière plastique, la charnière 36 étant formée d'une bande mince reliant le couvercle 30 et la plaque 23.

Le couvercle 30 peut comporter une partie principale 32 dont la face inférieure 33 vient au contact, ou à proximité immédiate, de la face supérieure de la plaque 23. La face inférieure du couvercle est également la face intérieure de celui-ci, c'est-à-dire la face tournée vers l'intérieur du bac 10, lorsque le couvercle 30 est en position fermée.

Selon une réalisation possible, et comme on le voit sur les figures 1 à 3, la partie principale 32 du couvercle 30 peut présenter, du côté de sa face inférieure 33, une zone en saillie qui vient se loger dans la zone en dépression 28 de la plaque 23 en position fermée du couvercle 30. De préférence la zone en saillie du couvercle 30 a une forme complémentaire de celle de la zone en dépression 28 de la plaque 23. Un joint d'étanchéité 34 peut être monté sur la face inférieure 33 du couvercle 30, typiquement autour de la zone en saillie de la partie principale 32, pour assurer l'étanchéité entre la platine 20 - plus spécifiquement la plaque 23 - et le couvercle 30 en position fermée.

Le couvercle 30 peut comporter un bras 38 reliant la partie principale 32 à la charnière 36 et une patte 35 s'étendant depuis la partie principale 32, à l'opposé de la charnière 36. La patte 35 permet de verrouiller le couvercle 30 en position fermée par rapport à la platine 20. A cet effet, la patte 35 peut comporter un trou 37 venant s'encliqueter dans un doigt 7 de la platine 20. Selon une réalisation possible, la plaque 23 de la platine 20 comporte une bordure 5 sensiblement verticale et périphérique, qui s'étend vers le haut (en position fermée de la plaque 23), le doigt 29 étant ménagé dans cette bordure 5. La bordure 5 peut être interrompue au niveau du bras 38 du couvercle 30, comme on le voit sur les figures 1 et 3.

De préférence, le couvercle 30 est verrouillé sur la platine 20 en position fermée, et la plaque 23 est verrouillée sur le cadre 21 en position fermée, de sorte que le déverrouillage ne puisse pas être réalisés par le praticien, mais uniquement par une entreprise spécialisée dans le traitement des déchets en question, par des moyens spécifiques.

La boîte 1 comprend de plus une cartouche 40, c'est-à-dire un petit récipient, qui est montée dans le bac 10 et qui est destiné à contenir un fluide de désinfection des déchets, généralement sous pression. Le fluide de désinfection est de préférence un produit désinfectant à large spectre, antiviral, antibactérien et fongicide. Il peut s'agir d'un gaz. Le volume intérieur de la cartouche est déterminé en fonction du volume du bac 10, afin que la quantité de fluide de désinfection garantisse une désinfection efficace.

La cartouche 40 comporte une enveloppe 41 et un actionneur mobile par rapport à l'enveloppe 41 entre une position inactive dans laquelle le fluide ne peut pas être expulsé de la cartouche 40 et une position active dans laquelle du fluide peut être expulsé.

Comme on le voit notamment sur les figures 6 (qui est une coupe selon le plan P1) et 11, l'enveloppe 41 peut comporter une paroi périphérique 42 sensiblement cylindrique, d'axe sensiblement vertical en position montée, et une paroi supérieure 43 présentant une forme générale de disque.

L'actionneur peut comporter un embout 44 muni d'un trou d'expulsion 45 du fluide, et fonctionner comme une valve d'aérosol. Ainsi, l'embout 44 peut être monté en partie supérieure de la cartouche 40 de façon mobile en translation verticale entre une position haute - c'est-à-dire en position inactive - et une position basse - c'est-à-dire en position active. De façon concrète, la paroi supérieure 43 de l'enveloppe 41 peut comporter une collerette 46 faisant saillie vers le haut et munie d'un filetage extérieur, dans lequel l'embout 44 vient se fixer au moyen d'une bague 47 vissée sur la collerette 46.

La cartouche 40 est de préférence disposée dans le bac 10 à proximité de ou contre la paroi périphérique 11 du bac 10. Lorsque la fenêtre 27 est sensiblement centrale, cette disposition évite que la cartouche 40 ne gêne l'introduction des déchets dans le bac 10. Par exemple, la cartouche 40 peut être disposé contre l'un des petits panneaux 14 de la paroi périphérique 11 du bac 10.

Selon l'invention, le couvercle 30 comprend des moyens d'activation de l'actionneur de la cartouche 40, c'est-à-dire ici de l'embout 44. La boîte 1 est configurée pour que, lorsque le couvercle 30 est en position ouverte, l'embout 44 soit en position haute, et pour que le passage du couvercle 30 à la position fermée provoque le passage de l'embout 44 à la position basse par l'action des moyens d'activation.

A cet effet, on peut prévoir que l'embout 44 soit agencé en regard d'un orifice 29 de la platine 20, de sorte que l'embout 44 et les moyens d'activation ménagés sur le couvercle 30 puissent coopérer. Dans la réalisation représentée à titre d'exemple, l'orifice 29 est ménagé dans la zone en dépression 28 de la plaque 23, à distance de la fenêtre 27.

En outre, comme on le voit sur les figures 2 et 5, les moyens d'activation peuvent comprendre un ergot 39 qui fait saillie sensiblement orthogonalement de la face inférieure 33 du couvercle 30. L'ergot 39 est agencé sur le couvercle 30 de sorte que, en position fermée du couvercle 30, il soit situé en regard de l'orifice 29 de la platine 20, comme cela est bien visible sur la figure 5.

Ainsi, l'orifice 29 de la platine 20 est en regard à la fois de l'ergot 39 du couvercle 30 (en position fermée du couvercle) et de l'embout 44 de la cartouche 40. On peut prévoir que l'orifice 29 présente des dimensions légèrement supérieures à celles de l'ergot 39 et/ou de l'embout 44, de sorte à assurer un guidage et un positionnement satisfaisant de ces deux éléments l'un par rapport à l'autre, pour garantir qu'ils viendront bien en contact et, en conséquence, que l'ergot 39 provoquera bien le passage de l'embout 44 en position basse.

On peut prévoir que, en position inactive, c'est-à-dire en position basse, l'embout 44 soit situé sous la face supérieure de la platine 20, voire même sous la face inférieure de la platine 20, plus précisément de la plaque 23. Ceci permet d'éviter que l'embout 44 ne soit abaissé, donc activé, de façon non intentionnelle. Avec cette configuration, en position fermée du couvercle, l'ergot 39 doit être logé dans l'orifice 29 de la platine 20, et même dépasser sous la plaque 23, de façon à pouvoir atteindre l'embout 44 et l'actionner. Ceci est illustré schématiquement sur la figure 6.

Ainsi, le praticien reçoit une boîte 1 prête à l'emploi, vide et désinfectée, avec la platine 20 fixée sur le bac 10, la plaque 23 fermée et verrouillée sur le cadre 21, et le couvercle 30 ouvert. La cartouche 40 contient une quantité adaptée de fluide de désinfection.

Une fois la boîte 1 pleine de déchets, le praticien ferme le couvercle 30. Ceci entraîne l'appui sur l'embout 44 de la cartouche 40 par l'ergot 39 du couvercle 30, et l'expulsion du fluide de désinfection par le trou d'expulsion 45. Le fluide reste contenu dans la boîte 1, puisque celle-ci est fermée de façon étanché, via les joints 24 et 34. Les déchets contenus dans la boîte 1 se trouvent ainsi désinfectés. De plus, le couvercle 30 est verrouillé sur la platine 20.

La boîte 1 est prise en charge par une entreprise spécialisée, qui va mener les opérations nécessaires au recyclage de la boîte.

A cet effet, l'ensemble comprenant la platine 20 et le couvercle 30 est désolidarisé du bac 10, généralement au moyen d'une machine spéciale. Puis, les déchets contenus dans le bac 10, et qui ne présentent désormais plus de risque infectieux, sont vidés. Ils seront ensuite traités comme des déchets ménagers. Le bac 10 est alors nettoyé et désinfecté, de façon à pouvoir être réutilisé. Il s'agit ensuite de remplacer la cartouche par une cartouche pleine de fluide de désinfection, ou de recharger la cartouche 40 en fluide de désinfection. L'ensemble comprenant la platine 20 et le couvercle 30 est à nouveau monté sur le bac 10. La boîte 1 est ainsi prête à être réutilisée par un praticien.

Selon un mode de réalisation, la cartouche 40 est montée dans le bac 10 de façon inamovible. Dans l'optique de rendre la boîte 1 réutilisable, il faut pouvoir recharger la cartouche 40 en fluide de désinfection, et la boîte 1 comporte donc des moyens de rechargement 100 de la cartouche 40 en fluide. Ces moyens de rechargement 100 sont de préférence ménagés sur la cartouche 40 et/ou sur le bac 10 et accessibles depuis l'extérieur de la boîte 1.

Comme on le voit sur la figure 6, un trou taraudé 17 peut être ménagé dans une paroi de l'enveloppe 41 et/ou du bac 10 pour recevoir les moyens de rechargement 100, ces derniers étant destinés à coopérer avec étanchéité avec la tête d'un système d'injection de fluide (non représenté) et mettre cette tête en communication avec le volume intérieur de la cartouche 40. Les moyens de rechargement 100 seront décrits plus loin avec davantage de détail.

A l'état prêt à l'emploi, la cartouche 40 forme un volume fermé destiné à contenir le fluide de désinfection. Pour résoudre le problème du moulage d'un tel volume fermé, notamment lorsque la cartouche est montée dans le bac 10 de façon inamovible, plusieurs solutions peuvent être envisagées. De façon générale, il s'agit de prévoir que, à l'état initial, l'enveloppe forme un volume ouvert, et que la boîte comporte au moins une paroi de fermeture qui, à l'état prêt à l'emploi, coopère avec l'enveloppe pour former un volume fermé apte à contenir le fluide. Cette paroi de fermeture peut être formée au moins en partie par une paroi du bac.

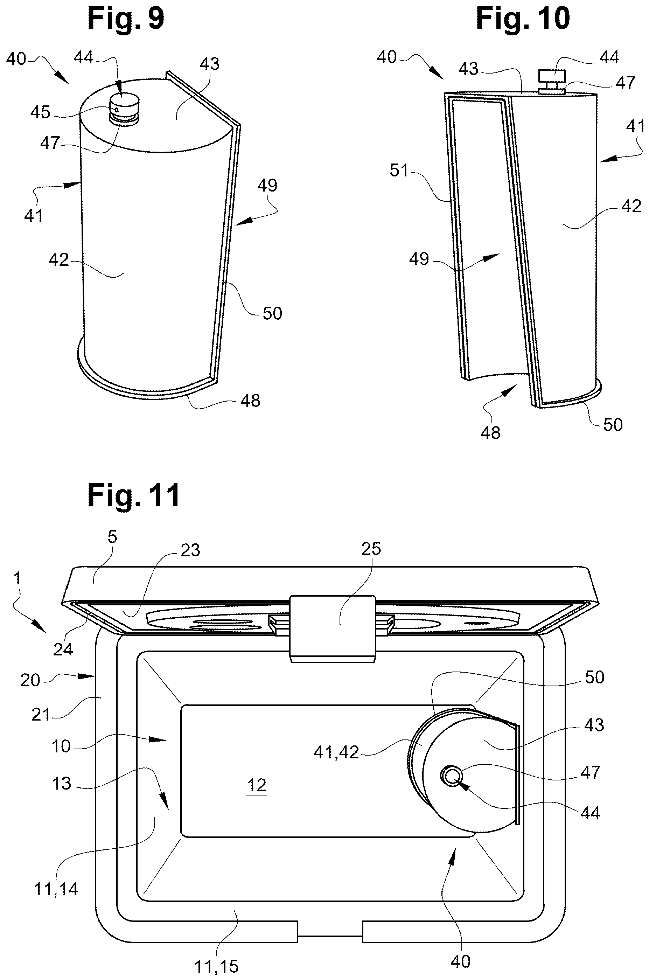

Selon un mode de réalisation, illustré tout particulièrement sur les figures 9 à 11, l'enveloppe 41 est une pièce distincte du bac 10 à l'état initial, c'est-à-dire avant d'être montée dans le bac 10. L'enveloppe 41 est par exemple réalisée par moulage d'une matière plastique. Une fois mise en place dans le bac 10, l'enveloppe 41 et le bac 10 peuvent former une seule et même pièce.

L'enveloppe 41 comporte une ouverture inférieure 48 et une ouverture latérale 49 et vient se fixer avec étanchéité contre la face intérieure du bac 10, de sorte que la paroi de fond 12 du bac 10 ferme l'ouverture inférieure 48 et que la paroi périphérique 11 du bac 10 ferme l'ouverture latérale 49 de l'enveloppe 41. Les ouvertures 48 et 49 peuvent être dans la continuité l'une de l'autre, c'est-à-dire former globalement une unique ouverture.

Par exemple, l'enveloppe 41 peut former un cylindre tronqué selon un plan sensiblement parallèle à la face de la paroi périphérique 11 du bac 10 contre laquelle l'enveloppe 41 est fixée. Ce plan peut être globalement parallèle à l'axe du cylindre, ou un peu incliné par rapport à celui-ci, typiquement si le bac 10 s'évase en direction de son ouverture 13. Dans la réalisation représentée, l'enveloppe 41 est fixée contre l'un des petits panneaux 14 du bac 10, mais d'autres agencements sont envisageables.

Ainsi, une zone A12 de la paroi de fond 12 du bac 10 forme la paroi inférieure de la cartouche 40, et une zone A11 de la paroi périphérique 11 du bac 10 forme une partie de la paroi périphérique 42 de l'enveloppe 41 de la cartouche 40. Ces zones A12, A11 forment une surépaisseur locale, vers l'intérieur du bac 10, par rapport aux zones adjacentes de la paroi de fond 12 et de la paroi périphérique 11 du bac 10, respectivement, comme cela est visible sur la figure 7. Ceci permet un guidage et un emboîtement de l'enveloppe 41 contre le bac 10. Le contour des ouvertures inférieure 48 et latérale 49 peut être pourvu d'un rebord 50, par exemple radial, venant se placer autour et de façon adjacente aux zones A12, A11, pour augmenter la surface d'appui entre l'enveloppe 41 et la face intérieure de la paroi périphérique 11 du bac 10 et permettre un assemblage de l'enveloppe 41 au bac 10, par exemple par thermosoudage. Un joint d'étanchéité 51 peut en outre être monté sur la face du rebord 50 qui viendra en contact avec le bac 10.

Par ailleurs, la zone A12 de la paroi de fond 12 du bac 10 qui forme le fond de la cartouche 40 possède avantageusement une partie 52 ayant une forme bombée vers l'intérieur du bac 10.

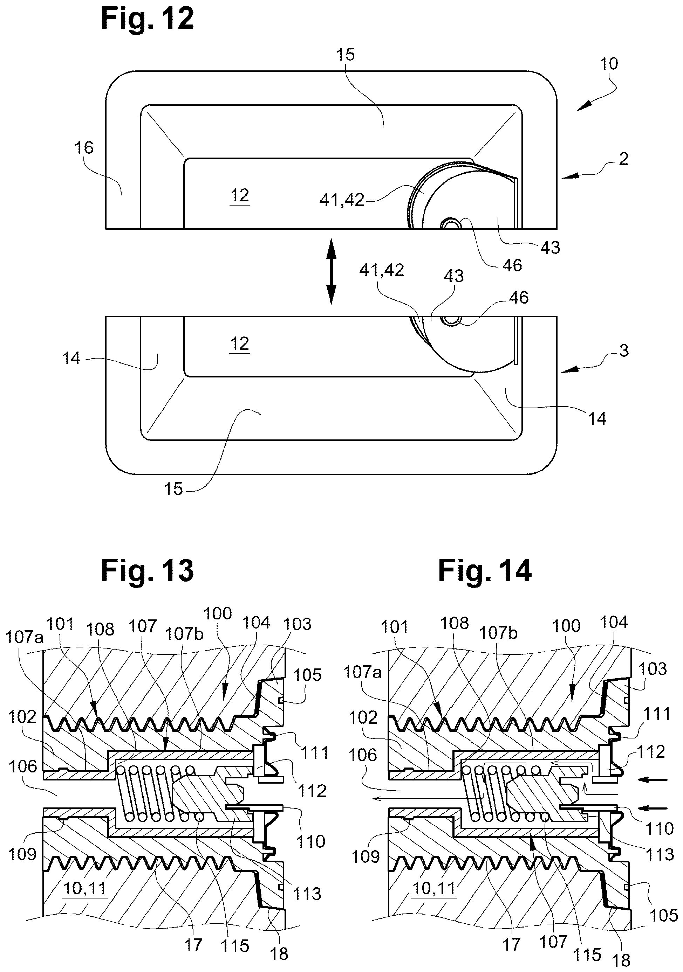

Selon un autre mode de réalisation, illustré sur la figure 12, le bac 10 est formé de plusieurs portions initialement distinctes, ici de deux portions 2, 3, qui correspondent par exemple chacune à une moitié de bac 10 située d'un côté du plan vertical longitudinal médian P1. Chaque portion 2, 3 du bac 10 constitue à la fois une portion de bac et une portion d'enveloppe. Plus précisément, chaque portion 2, 3 peut former l'un des grands panneaux 15 de la paroi périphérique 11 du bac 10, ainsi qu'une moitié de chacun des petits panneaux 14 et une moitié de la paroi de fond 12 et, de plus, une moitié de l'enveloppe 41 (c'est-à-dire de la paroi périphérique 42 et de la paroi supérieure 43). Chaque portion 2, 3 peut être réalisée d'une seule pièce par moulage.

Ces deux portions 2, 3 sont assemblées avec étanchéité pour former le bac 10 et la cartouche 40 intégrée au bac 10, à l'état prêt à l'emploi. L'assemblage peut par exemple être réalisé par thermosoudage.

Avec ce mode de réalisation, la paroi de fermeture de la cartouche, qui est ouverte à l'état initial, est formée en partie par une zone d'une paroi du bac, et en partie par une demi-enveloppe de la cartouche.

Là encore, la zone de la paroi de fond 12 du bac 10 qui forme le fond de la cartouche 40 possède de préférence une partie ayant une forme bombée vers l'intérieur du bac 10.

On se réfère à présent aux figures 13 et 14, qui illustrent plus en détail un mode de réalisation des moyens de rechargement 100 de la cartouche 40 en fluide.

Selon ce mode de réalisation, les moyens de rechargement 100 comportent un corps 101 possédant :
- une partie cylindrique filetée 102 venant s'insérer dans le trou taraudé 17 qui est ménagé dans une paroi du bac 10 (telle que la paroi périphérique 11 dans l'exemple illustré) et communique avec le volume intérieur de la cartouche 40 ;
- et une collerette 103 qui peut venir se loger dans une cavité 18 de ladite paroi du bac 10, entourant le trou taraudé 17 du côté extérieur du bac 10. La cavité 18 a de préférence une forme correspondante à celle de la collerette 103, de sorte qu'à l'état monté le corps 101 affleure sensiblement la paroi du bac 10 dans laquelle il est placé, comme on le voit sur les figures 13 et 14. Un joint d'étanchéité 104 peut être prévu entre la collerette 103 et la cavité 18.

En outre, la collerette 103 peut comporter une encoche 105 dans laquelle peut être inséré un outil de serrage pour sécuriser le montage du corps 101 dans la paroi du bac 10.

Le corps 101 peut être réalisé dans le même matériau que le bac 10, par exemple en matière plastique.

Le corps 101 possède un canal 106 intérieur, de préférence axial, débouchant aux deux extrémités, c'est-à-dire à l'intérieur et à l'extérieur du bac 10. Ce canal 106 peut présenter une première portion située du côté extérieur du bac 10 et possédant un premier diamètre, et une deuxième portion située du côté intérieur du bac 10 et possédant un deuxième diamètre inférieur au premier diamètre. Par exemple, la première portion peut s'étendre sur environ les deux tiers de la longueur total du canal 106.

Un manchon 107 est monté dans le canal 106. Le manchon 107 possède une forme externe correspondant à la forme interne du canal 106. Ainsi, le manchon 107 peut présenter une première portion 107a située du côté extérieur du bac 10 et possédant un premier diamètre, et une deuxième portion 107b située du côté intérieur du bac 10 et possédant un deuxième diamètre inférieur au premier diamètre. Entre ces deux portions 107a, 107b est donc défini un épaulement 108.

Pour assurer la solidité du montage, il peut être prévu que le manchon 107 comporte un bourrelet externe 109, par exemple dans la deuxième portion 107b, inséré dans une gorge interne du canal 106. Le manchon peut être réalisé en métal, par exemple en acier inoxydable.

Le manchon 107 définit un logement dans lequel est reçu le mécanisme qui permet :
- lorsqu'il est à l'état actif, de faire entrer le fluide de désinfection, afin de recharger la cartouche 40 (figure 14) ;
- et lorsqu'il est à l'état inactif, d'assurer l'étanchéité entre l'intérieur et l'extérieur du bac 10 (figure 13).

Dans l'exemple de réalisation illustré, ce mécanisme comporte un embout 110 monté dans le canal 106 au voisinage de l'extrémité extérieure de ce canal, au moyen d'une bague de sertissage 111. Un joint d'étanchéité 112 annulaire monté dans le corps 101 est disposé autour de l'embout 110, et permet d'assurer l'étanchéité avec la tête du système d'injection lors de la recharge.

Le mécanisme comporte de plus une pièce 113 d'ouverture / fermeture, qui est mobile axialement entre une position de fermeture (illustrée sur la figure 13) et une position d'ouverture (figure 14). Un ressort 115, en appui sur l'épaulement 108, pousse la pièce 113 vers sa position d'ouverture, dans laquelle elle est en butée contre le joint d'étanchéité 112. et empêche le passage de fluide de désinfection, que ce soit vers l'intérieur ou vers l'extérieur du bac 10.

Pour recharger la cartouche 40, on introduit la tête du système d'injection de fluide dans l'embout 110, ce qui a pour effet de déplacer la pièce 113 vers sa position d'ouverture, à l'encontre de la force exercée par le ressort 115. Alors, comme illustré sur la figure 14, le fluide peut circuler dans le canal 106 jusqu'à l'intérieur du bac 10 et au volume intérieur de la cartouche 40.

Lorsque la cartouche 40 est rechargée, la tête du système d'injection est ôtée de l'embout 110 et, sous l'effet du ressort 115, la pièce 113 est ramenée à sa position de fermeture.

Ainsi, l'invention apporte une amélioration déterminante à la technique antérieure, en permettant la réutilisation de ce type de boîte, et donc la diminution significative de la quantité de déchets.

## Revendications

1. Boîte (1) destinée à recevoir des déchets, en particulier des déchets médicaux, comprenant :
- un bac (10) comportant une paroi périphérique (11), une paroi de fond (12), et une ouverture (13) opposée à la paroi de fond (12) ;
- une platine (20) fixée au bac (10), couvrant sensiblement l'ouverture (13) du bac (10), et comportant au moins une fenêtre (27) pour l'introduction de déchets dans le bac (10) ; par « couvrant sensiblement » on entend que la platine couvre entièrement ladite ouverture (13) à l'exception de la fenêtre (27);
- un couvercle (30) monté sur la platine (20) de façon mobile entre une position ouverte, dans laquelle la fenêtre (27) de la platine (20) est accessible, et une position fermée, dans laquelle le couvercle (30) obture ladite fenêtre (27) ;
- une cartouche (40) apte à contenir un fluide de désinfection des déchets, la cartouche (40) étant montée dans le bac (10) et comportant :
- une enveloppe (41) ;
- un trou (45) d'expulsion du fluide ;
- un actionneur (44) mobile par rapport à l'enveloppe (41) entre une position inactive dans laquelle le fluide ne peut pas être expulsé de la cartouche (40) et une position active dans laquelle du fluide peut être expulsé par le trou (45) d'expulsion ;
le couvercle (30) comprenant des moyens d'activation (39) de l'actionneur (44) de la cartouche (40) ;
la boîte (1) étant configurée pour que, lorsque le couvercle (30) est en position ouverte, l'actionneur (44) soit en position inactive, et pour que le passage du couvercle (30) à la position fermée provoque le passage de l'actionneur (44) à la position active par l'action des moyens d'activation (39),
**caractérisée en ce que** la cartouche (40) est montée dans le bac (10) de façon inamovible, et **en ce que** la boîte (1) comporte des moyens de rechargement (100) de la cartouche (40) en fluide, les moyens de rechargement (100) étant ménagés sur la cartouche (40) et/ou sur le bac (10), et étant accessibles depuis l'extérieur de la boîte (1).

2. Boîte selon la revendication 1, **caractérisée en ce que** les moyens d'activation comprennent un ergot (39) faisant saillie orthogonalement de la face intérieure (33) du couvercle (30) et **en ce que** la platine (20) comprend un orifice (29) qui, lorsque le couvercle (30) est en position fermée, est situé en regard de l'ergot du couvercle (30) et de l'actionneur (44) de la cartouche (40), de sorte que l'ergot (39) puisse coopérer avec l'actionneur (44) pour le faire passer à sa position active.

3. Boîte selon la revendication 2, **caractérisée en ce que**, en position fermée du couvercle (30), l'ergot (39) est au moins en partie logé dans l'orifice (29) de la platine (20), l'orifice (29) de la platine (20) présentant de préférence des dimensions supérieures à celles de l'ergot (39).

4. Boîte selon l'une des revendications 1 à 3, **caractérisée en ce que** l'actionneur comprend un embout (44) monté en partie supérieure de la cartouche (40) de façon mobile en translation verticale, entre une position haute, correspondant à la position inactive, et une position basse, correspondant à la position active, l'embout (44) étant agencé en regard d'un orifice (29) de la platine (20) de sorte à pouvoir coopérer avec les moyens d'activation (39) ménagés sur le couvercle (30).

5. Boîte selon la revendication 4, **caractérisée en ce que**, en position inactive, l'embout (44) est situé sous la face supérieure de la platine (20).

6. Boîte selon l'une des revendications 1 à 5, **caractérisée en ce que** les moyens de rechargement (100) comportent une pièce (113) qui est montée dans un trou (17) du bac (10), en regard de la cartouche (40), de façon mobile entre une position ouverte, permettant l'entrée dans la cartouche (40) du fluide de désinfection des déchets, et une position fermée assurant l'étanchéité audit fluide entre l'intérieur et l'extérieur du bac (10).

7. Boîte selon l'une des revendications 1 à 6, **caractérisée en ce que** la cartouche (40) est disposée dans le bac (10) à proximité de ou contre la paroi périphérique (11) du bac (10).

8. Boîte selon l'une des revendications 1 à 7, **caractérisée en ce que**, à l'état initial, l'enveloppe (41) forme un volume ouvert, et **en ce que** la boîte (1) comporte au moins une paroi de fermeture qui, à l'état prêt à l'emploi, coopère avec l'enveloppe (41) pour former un volume fermé apte à contenir le fluide.

9. Boîte selon la revendication 8, **caractérisée en ce que** ladite paroi de fermeture est formée au moins en partie par une paroi (11, 12) du bac (10).

10. Boîte selon l'une des revendications 1 à 9, **caractérisée en ce que** l'enveloppe (41) est une pièce distincte du bac (10), à l'état initial, qui comporte une ouverture inférieure (48) et une ouverture latérale (49), ladite enveloppe (41) venant se fixer avec étanchéité contre la face intérieure du bac (10), de sorte que la paroi de fond (12) du bac (10) forme le fond de la cartouche (40) et que la paroi périphérique du bac (10) ferme l'ouverture latérale de l'enveloppe (41).

11. Boîte selon la revendication 10, **caractérisée en ce que** la zone (A12) de la paroi de fond (12) du bac (10) qui forme le fond de la cartouche (40) et la zone (A11) de la paroi périphérique (11) du bac (10) qui ferme l'ouverture latérale (49) de l'enveloppe (41) forment une surépaisseur locale, vers l'intérieur du bac (10), par rapport aux zones adjacentes de la paroi de fond (12) et de la paroi périphérique (11) du bac (10), respectivement, permettant un guidage et un emboîtement de l'enveloppe (41) contre le bac (10).

12. Boîte selon l'une des revendications 1 à 9, **caractérisée en ce qu'**elle comprend au moins deux portions (2, 3) initialement distinctes, chaque portion (2, 3) constituant une portion de bac (10) et une portion d'enveloppe (41) et étant de préférence réalisée d'une seule pièce par moulage, lesdites deux portions (2, 3) étant assemblées avec étanchéité pour former le bac (10) et la cartouche (40) intégrée au bac (10).

13. Boîte selon l'une des revendications 10 à 12, **caractérisée en ce que** la zone de la paroi de fond (12) du bac (10) qui forme le fond de la cartouche (40) possède une partie (52) ayant une forme bombée vers l'intérieur du bac (10).

14. Procédé de recyclage d'une boîte (1) selon l'une des revendications précédentes, le couvercle (30) étant en position fermée, le procédé étant **caractérisé en ce qu'**il comprend les étapes suivantes :
- désolidariser du bac (10) l'ensemble comprenant la platine (20) et le couvercle (30) ;
- vider les déchets contenus dans le bac (10) ;
- nettoyer et désinfecter le bac (10) ;
- recharger la cartouche (40) en fluide de désinfection, par lesdits moyens de rechargement (17), depuis l'extérieur de la boîte ;
- monter à nouveau sur le bac (10) l'ensemble comprenant la platine (20) et le couvercle (30).

## Patentansprüche

1. Kasten (1) zur Aufnahme von Abfällen, insbesondere medizinischen Abfällen, umfassend:
- eine Wanne (10), die eine Umfangswand (11), eine Bodenwand (12) und eine der Bodenwand (12) gegenüberliegende Öffnung (13) aufweist;
- eine Platte (20), die an der Wanne (10) befestigt ist, die Öffnung (13) der Wanne (10) im Wesentlichen abdeckt und mindestens ein Fenster (27) zum Einführen von Abfällen in die Wanne (10) aufweist; unter "im Wesentlichen abdeckt" ist zu verstehen, dass die Platte die Öffnung (13) mit Ausnahme des Fensters (27) vollständig abdeckt;
- einen Deckel (30), der beweglich zwischen einer offenen Position, in der das Fenster (27) der Platte (20) zugänglich ist, und einer geschlossenen Position, in der der Deckel (30) das Fenster (27) verschließt, auf der Platte (20) montiert ist;
- eine Kartusche (40), die geeignet ist, ein Fluid zur Desinfektion der Abfälle zu enthalten, wobei die Kartusche (40) in der Wanne (10) montiert ist und Folgendes aufweist:
- eine Hülle (41);
- ein Loch (45) zum Ausstoßen des Fluids;
- einen Aktuator (44), der gegenüber der Hülle (41) zwischen einer inaktiven Position, in der das Fluid nicht aus der Kartusche (40) ausgestoßen werden kann, und einer aktiven Position, in der Fluid aus dem Loch (45) zum Ausstoßen ausgestoßen werden kann, beweglich ist;
wobei der Deckel (30) Aktivierungsmittel (39) für den Aktuator (44) der Kartusche (40) umfasst;
wobei der Kasten (1) so eingerichtet ist, dass, wenn sich der Deckel (30) in der geöffneten Position befindet, der Aktuator (44) in der inaktiven Position ist, und dass der Übergang des Deckels (30) in die geschlossene Position den Übergang des Aktuators (44) in die aktive Position durch die Betätigung der Aktivierungsmittel (39) bewirkt,
**dadurch gekennzeichnet, dass** die Kartusche (40) in der Wanne (10) unbeweglich montiert ist, und dass der Kasten (1) Nachfüllmittel (100) für die Kartusche (40) mit Fluid aufweist, wobei die Nachfüllmittel (100) an der Kartusche (40) und/oder an der Wanne (10) ausgebildet sind und von der Außenseite des Kastens (1) zugänglich sind.

2. Kasten nach Anspruch 1, **dadurch gekennzeichnet, dass** die Aktivierungsmittel eine Nase (39) umfassen, die orthogonal aus der Innenseite (33) des Deckels (30) herausragt, und dass die Platte (20) eine Öffnung (29) umfasst, die, wenn der Deckel (30) in geschlossener Position ist, gegenüber der Nase des Deckels (30) und des Aktuators (44) der Kartusche (40) liegt, so dass die Nase (39) mit dem Aktuator (44) zusammenwirken kann, um ihn in seine aktive Position zu bringen.

3. Kasten nach Anspruch 2, **dadurch gekennzeichnet, dass** in geschlossener Position des Deckels (30) die Nase (39) mindestens teilweise in der Öffnung (29) der Platte (20) untergebracht ist, wobei die Öffnung (29) der Platte (20) vorzugsweise größere Abmessungen besitzt als die der Nase (39).

4. Kasten nach einem der Ansprüche 1 bis 3, **dadurch gekennzeichnet, dass** der Aktuator ein Endstück (44) umfasst, dass im oberen Teil der Kartusche (40) beweglich in vertikaler Translation zwischen einer oberen Position, die der inaktiven Position entspricht, und einer unteren Position, die der aktiven Position entspricht, montiert ist, wobei das Endstück (44) gegenüber einer Öffnung (29) der Platte (20) angeordnet ist, so dass es mit den am Deckel (30) vorgesehenen Aktivierungsmitteln (39) zusammenwirken kann.

5. Kasten nach Anspruch 4, **dadurch gekennzeichnet, dass** sich in der inaktiven Position das Endstück (44) unter der Oberseite der Platte (20) befindet.

6. Kasten nach einem der Ansprüche 1 bis 5, **dadurch gekennzeichnet, dass** die Nachfüllmittel (100) ein Teil (113) aufweisen, das in einem Loch (17) der Wanne (10) gegenüber der Kartusche (40) beweglich zwischen einer offenen Position, die das Eindringen des Fluids zur Desinfektion der Abfälle in die Kartusche (40) ermöglicht, und einer geschlossenen Position, die die Abdichtung dieses Fluids zwischen dem Inneren und dem Äußeren der Wanne (10) gewährleistet, montiert ist.

7. Kasten nach einem der Ansprüche 1 bis 6, **dadurch gekennzeichnet, dass** die Kartusche (40) in der Wanne (10) in der Nähe oder an der Umfangswand (11) der Wanne (10) angeordnet ist.

8. Kasten nach einem der Ansprüche 1 bis 7, **dadurch gekennzeichnet, dass** die Hülle (41) im Ausgangszustand ein offenes Volumen bildet, und dass der Kasten (1) mindestens eine Verschlusswand aufweist, die im gebrauchsfertigen Zustand mit der Hülle (41) zusammenwirkt, um ein geschlossenes Volumen zu bilden, das zur Aufnahme des Fluids geeignet ist.

9. Kasten nach Anspruch 8, **dadurch gekennzeichnet, dass** die Verschlusswand mindestens teilweise durch eine Wand (11, 12) der Wanne (10) gebildet ist.

10. Kasten nach einem der Ansprüche 1 bis 9, **dadurch gekennzeichnet, dass** die Hülle (41) im Ausgangszustand ein von der Wanne (10) getrenntes Teil ist, das eine untere Öffnung (48) und eine seitliche Öffnung (49) aufweist, wobei die Hülle (41) dicht an der Innenseite der Wanne (10) befestigt wird, so dass die Bodenwand (12) der Wanne (10) den Boden der Kartusche (40) bildet und die Umfangswand der Wanne (10) die seitliche Öffnung der Hülle (41) verschließt.

11. Kasten nach Anspruch 10, **dadurch gekennzeichnet, dass** der Bereich (A12) der Bodenwand (12) der Wanne (10), der den Boden der Kartusche (40) bildet, und der Bereich (A11) der Umfangswand (11) der Wanne (10), der die seitliche Öffnung (49) der Hülle (41) verschließt, eine lokale Verdickung zum Inneren der Wanne (10) hin in Bezug auf die benachbarten Bereiche der Bodenwand (12) beziehungsweise der Umfangswand (11) der Wanne (10) bilden, wodurch eine Führung und ein Ineinandergreifen der Hülle (41) mit der Wanne (10) ermöglicht wird.

12. Kasten nach einem der Ansprüche 1 bis 9, **dadurch gekennzeichnet, dass** er mindestens zwei anfänglich getrennte Abschnitte (2, 3) umfasst, wobei jeder Abschnitt (2, 3) einen Wannenabschnitt (10) und einen Hüllenabschnitt (41) bildet und vorzugsweise aus einem einzigen Stück pro Gussstück hergestellt ist, wobei die beiden Abschnitte (2, 3) dicht zusammengefügt sind, um die Wanne (10) und die in die Wanne (10) integrierte Kartusche (40) zu bilden.

13. Kasten nach einem der Ansprüche 10 bis 12, **dadurch gekennzeichnet, dass** der Bereich der Bodenwand (12) der Wanne (10), der den Boden der Kartusche (40) bildet, ein Teil (52) mit einer zum Inneren der Wanne (10) hin gewölbten Form besitzt.

14. Recyclingverfahren für einen Kasten (1) nach einem der vorhergehenden Ansprüche, wobei sich der Deckel (30) in geschlossener Position befindet, wobei das Verfahren **dadurch gekennzeichnet ist, dass** es die folgenden Schritte umfasst:
- Lösen der Baugruppe umfassend die Platte (20) und den Deckel (30) von der Wanne (10);
- Leeren der in der Wanne (10) enthaltenen Abfälle;
- Reinigen und Desinfizieren der Wanne (10);
- Nachfüllen der Kartusche (40) mit Fluid zur Desinfektion über die Nachfüllmittel (17) von außerhalb des Kastens;
- erneutes Montieren der Baugruppe umfassend die Platte (20) und den Deckel (30) auf die Wanne (10).

## Claims

1. A box (1) for receiving wastes, in particular medical wastes, comprising:
- a receptacle (10) including a peripheral wall (11), a bottom wall (12), and an opening (13) opposite to the bottom wall (12);
- a plate (20) fastened to the receptacle (10), substantially covering the opening (13) of the receptacle (10), and including at least one aperture (27) for introducing wastes into the receptacle (10);
by "substantially covering", it should be understood that the plate completely covers said opening (13), with the exception of the aperture (27);
- a cover (30) mounted on the plate (20) movably between an open position, in which the aperture (27) of the plate (20) is accessible, and a closed position, in which the cover (30) seals said aperture (27);
- a cartridge (40) adapted to contain a waste disinfection fluid, the cartridge (40) being mounted in the receptacle (10) and including:
- a casing (41);
- a fluid expulsion hole (45);
- an actuator (44) movable relative to the casing (41) between an inactive position in which the fluid cannot be expelled from the cartridge (40) and an active position in which fluid can be expelled through the expulsion hole (45);
the cover (30) comprising means (39) for activating the actuator (44) of the cartridge (40);
the box (1) being configured so that, when the cover (30) is in the open position, the actuator (44) is in the inactive position, and so that the switch of the cover (30) into the closed position causes the switch of the actuator (44) into the active position by the action of the activation means (39),
**characterized in that** the cartridge (40) is mounted in the receptacle (10) in a non-removable manner, and **in that** the box (1) includes means (100) for recharging the cartridge (40) with fluid, the recharging means (100) being formed on the cartridge (40) and/or on the receptacle (10), and being accessible from outside the box (1).

2. The box according to claim 1, **characterized in that** the activation means comprise a lug (39) projecting orthogonally from the internal face (33) of the cover (30) and **in that** the plate (20) comprises an orifice (29) which, when the cover (30) is in the closed position, is located opposite the lug of the cover (30) and the actuator (44) of the cartridge (40), so that the lug (39) could cooperate with the actuator (44) to make it switch into its active position.

3. The box according to claim 2, **characterized in that**, in the closed position of the cover (30), the lug (39) is at least partially accommodated in the orifice (29) of the plate (20), the orifice (29) of the plate (20) preferably having dimensions larger than those of the lug (39).

4. The box according to one of claims 1 to 3, **characterized in that** the actuator comprises an endpiece (44) mounted in the upper portion of the cartridge (40) movable in vertical translation, between a high position, corresponding to the inactive position, and a low position, corresponding to the active position, the endpiece (44) being arranged opposite an orifice (29) of the plate (20) so as to be able to cooperate with the activation means (39) formed on the cover (30).

5. The box according to claim 4, **characterized in that**, in the inactive position, the endpiece (44) is located beneath the upper face of the plate (20).

6. The box according to one of claims 1 to 5, **characterized in that** the recharging means (100) include a part (113) which is mounted in a hole (17) of the receptacle (10), opposite the cartridge (40), movably between an open position, enabling entry of the waste disinfection fluid into the cartridge (40), and a closed position ensuring sealing to said fluid between the inside and the outside of the receptacle (10).

7. The box according to one of claims 1 to 6, **characterized in that** the cartridge (40) is disposed in the receptacle (10) proximate to or against the peripheral wall (11) of the receptacle (10).

8. The box according to one of claims 1 to 7, **characterized in that**, in the initial state, the casing (41) forms an open volume, and **in that** the box (1) includes at least one closure wall which, in the ready-to-use state, cooperates with the casing (41) to form a closed volume capable of containing the fluid.

9. The box according to claim 8, **characterized in that** said closure wall is formed at least partially by a wall (11, 12) of the receptacle (10).

10. The box according to one of claims 1 to 9, **characterized in that** the casing (41) is a part distinct from the receptacle (10), in the initial state, which includes a lower opening (48) and a lateral opening (49), said casing (41) being sealingly fixed against the internal face of the receptacle (10), so that the bottom wall (12) of the receptacle (10) forms the bottom of the cartridge (40) and the peripheral wall of the receptacle (10) seals the lateral opening of the casing (41).

11. The box according to claim 10, **characterized in that** the area (A12) of the bottom wall (12) of the receptacle (10) that forms the bottom of the cartridge (40) and the area (A11) of the peripheral wall (11) of the receptacle (10) that seals the lateral opening (49) of the casing (41) form a local extra thickness, towards the inside of the receptacle (10), with respect to the adjacent areas of the bottom wall (12) and of the peripheral wall (11) of the receptacle (10), respectively, enabling guidance and nesting of the casing (41) against the receptacle (10).

12. The box according to one of claims 1 to 9, **characterized in that** it comprises at least two initially distinct portions (2, 3), each portion (2, 3) constituting a receptacle portion (10) and a casing portion (41) and being preferably made in one-piece by molding, said two portions (2, 3) being sealingly assembled together to form the receptacle (10) and the cartridge (40) integrated into the receptacle (10).

13. The box according to one of claims 10 to 12, **characterized in that** the area of the bottom wall (12) of the receptacle (10) that forms the bottom of the cartridge (40) has a portion (52) having a shape cambered towards the inside of the receptacle (10).

14. A method for recycling a box (1) according to one of the preceding claims, the cover (30) being in the closed position, the method being **characterized in that** it comprises the following steps:
- detaching the assembly comprising the plate (20) and the cover (30) from the receptacle (10);
- emptying the wastes contained in the receptacle (10);
- cleaning and disinfecting the receptacle (10);
- recharging the cartridge (40) with disinfection fluid, by said recharging means (17), from outside the box;
- mounting the assembly comprising the plate (20) and the cover (30) again on the receptacle (10).
